Europäisches Patentamt

European Patent Office

Office européen des brevets

(11) Publication number: 0 361 977
A2

# EUROPEAN PATENT APPLICATION

(21) Application number: 89310021.4

(22) Date of filing: 29.09.89

(51) Int. Cl.5: C07K 5/10 , A61K 37/02

(30) Priority: 30.09.88 US 252505

(43) Date of publication of application:
04.04.90 Bulletin 90/14

(84) Designated Contracting States:
AT BE CH DE ES FR GB GR IT LI LU NL SE

(71) Applicant: IMMUNOBIOLOGY RESEARCH
INSTITUTE, INC.
Route 22 East P.O. Box 999
Annandale New Jersey 08801-0999(US)

(72) Inventor: Heavner, George
Box 73B Summer Road
Flemington New Jersey 08822(US)
Inventor: Audhya, Tapan
473 Foothill Road
Bridgewater New Jersey 08807(US)
Inventor: Goldstein, Gideon
30 Dorison Drive
Short Hills New Jersey 07078(US)

(74) Representative: Hale, Stephen Geoffrey et al
J.Y. & G.W. Johnson Furnival House 14/18
High Holborn
London WC1V 6DE(GB)

(54) Peptides having T cell suppressor activity.

(57) Peptides having the biological activity of the molecule human thymopentin, namely the ability to increase cGMP activity in human T-cell line CEM, have the formula

R-Arg-X-Asp-Y-NR$^1$R$^2$

wherein

R is H, lower alkyl, acetyl, formyl lower alkanoyl;

X is Pro, dehydro-Pro, hydroxyl-Pro, D-Lys, Aib or Lys;

Y is Gly or a D or L form of an amino acid selected from Val, Ala, Ile, Leu, Asp, Glu, Gln or Lys;

R$^1$ is H and R$^2$ is lower alkyl or alkenyl having 1 to 10 carbon atoms, cyclic alkyl or alkenyl having 4 to 8 carbon atoms, aryl or NHR$^3$, where R$^3$ is a lower alkyl having 1 to 10 carbon atoms, cyclic alkyl or alkenyl having 4 to 8 carbon atoms, or aryl; or

R$^1$ and R$^2$ together comprise a methylene chain of 4 to 7 carbon atoms.

These peptides can be used in pharmaceutical compositions for regulating a deficiency or excess of T-cell function in human subjects.

## PEPTIDES HAVING T CELL SUPPRESSOR ACTIVITY

The present invention relates generally to synthetic peptides capable of stimulating suppressor T cell activity. More particularly, the peptides of the present invention are tetrapeptides having amide substitutions on the carboxy-terminus, which are based on the molecule thymopoietin.

Background of the Invention

The immunomodulatory proteins, thymopoietin and thysplenin (formerly "splenin"), have been isolated from bovine and human thymus and spleen, respectively. Additionally, small peptides have been chemically synthesized which mimic the biological·activity of thymopoietin and thysplenin have been further modified to be provided with additional attributes such as resistance to enzymatic action. See, e.g. U. S. Patent 4,505,853 and co-owned European Patent Application No. 89304998.1.

A large body of articles and patents have now been published relating to such proteins and synthesized peptides. U. S. Patent No. 4,190,646 discloses the pentapeptide thymopentin which is the active site of thymopoietin and has the sequence Arg-Lys-Asp-Val-Tyr, as well as peptide compositions in which various substitutions are made on the amino and/or carboxy termini of this pentapeptide. Both thymopoietin and thymopentin induce biological changes in two human T-cell lines, CEM and MOLT-4, thereby indicating a role in stimulating both the suppressor and helper activities of T cells. No analogs of thymopentin shorter than pentapeptides (5 amino acids in sequence) were found to be active on CEM cells.

European Patent Application No. 292,302 discloses a 48 amino acid immunomodulatory protein, splenin, (hereafter referred to as "thysplenin") isolated from human spleen. The active site of bovine thysplenin, called SP-5, which spans amino acid residues 32-36 thereof and has the sequence Arg-Lys-Glu-Val-Tyr stimulates helper T cell activity in vivo in mice. Human thysplenin is thus expected to exhibit analogous biological activity in humans. Human thysplenin was described in the above-identified application as inducing elevation of intracellular cGMP in the human T cell line MOLT-4. In the SN 56186 application the active site of the human sequence was disclosed as Arg-Lys-Ala-Val-Tyr.

Thysplenin, unlike thymopoietin, does not produce changes in biological activity of CEM cells. Thus thysplenin is implicated in the stimulation of T cell helper activity, not T cell suppressor activity. See also U.S. Patents 4,190,646; 4,261,886; 4,361,673; 4,420,424; and 4,629,723. Reference is made to the above-described patents, applications and articles for a discussion of other background material and the biological processes involved in the present invention.

U. S. Patent No. 4,428,938 by Kisfaludy et al, issued January 31, 1984, discloses certain peptides affecting immune regulation. Among such peptides are the following tetrapeptides:
Arg-Lys-Asp-Val
Arg-Lys-Asn-Val
Arg-Lys-Ala-Val
Arg-Lys-Asp-Ala
Arg-Lys-Asp-Ile
Arg-Lys-Glu-Val
Glp-Arg-Lys-Asp
The '938 patent generally recites that the salts, amides, lower alkyl esters and protected derivatives of these sequences are also capable of effecting immune regulation. However, no particular amide of these sequences is identified with specificity therein. Methods for using these sequences to treat immunological disorders due to thymic deficiencies are also discussed. In this patent the peptides were tested for activity in an in vitro E rosette assay.

Applicants' co-owned European Patent Application 89304998.1 disclosed a series of thysplenin peptide analogs capable of inducing biological activity in the MOLT-4 T cell line. Unlike the thymopentin analogs previously reported, peptides smaller than five amino acids in length and related to thysplenin were active in inducing T cell helper activity in MOLT-4 cells, as well as pentapeptide analogs of thysplenin described herein. Several tetrapeptides described therein included:
Arg-Pro-Asp-Val
Arg-Pro-Asp-Val-NH$_2$
Formyl-Arg-Pro-Asp-Val
Acetyl-Arg-Pro-Asp-Val
Acetyl-Arg-Pro-Asp-Val-NH$_2$
Acetyl-Arg-Pro-Ala-Val-NH$_2$
Acetyl-Arg-Pro-D-beta-Asp-Val-NH$_2$
Acetyl-Arg-Pro-Glu-Val-NH$_2$
Acetyl-Arg-Pro-Glu-Val
Acetyl-Arg-Aib-Ala-Val-NH$_2$
Acetyl-Arg-Aib-Glu-Val-NH$_2$
Acetyl-Arg-Pro-Asp-Glu-NH$_2$.
These peptides were found to mimic the action of thysplenin in the MOLT-4 assay, but could not mimic thymopentin's activity in the CEM cell line assay.

There remains a need in the art for additional peptides which are useful in stimulating the suppressor and helper activities of immune system of humans for a variety of T cell deficient conditions.

## Summary of the Invention

It has surprisingly been discovered that a number of tetrapeptides containing carboxy terminal substituted amides possess the T cell suppressor activity characteristic of thymopentin in cyclic GMP assays on CEM cells. This discovery is surprising because the same tetrapeptides with a free carboxy-terminus or amidated with different C terminal amide groups do not produce such T cell suppressor activity. Several of the tetrapeptide sequences have been previously identified, e.g. in the U. S. patent 4,428,938, which with a free carboxy-terminus do not produce any T cell suppressor activity. Yet, when certain amide substitutions are placed on the C-terminus according to this invention, these inactive peptides evince T cell suppressor activity. Such activity is not predictable by any of the prior art.

Therefore, in one aspect the present invention provides the following amidated peptides of formula

R-Arg-X-Asp-Y-NR$^1$R$^2$

wherein

R is H, lower alkyl, acetyl, formyl, lower alkanoyl; X is Pro, dehydro-Pro, hydroxyl-Pro, D-Lys, Aib or Lys; Y is Glu or a D or L form of an amino acid selected from Val, Ala, Ile, Leu, Asp, Glu, Gln, or Lys; R$^1$ is H and R$^2$ is lower alkyl or alkenyl having 1 to 10 carbon atoms, cyclic alkyl or alkenyl having 4 to 8 carbon atoms, aryl or NHR$_3$, where R$_3$ is a lower alkyl having 1 to 10 carbon atoms, cyclic alkyl or alkenyl having 4 to 8 carbon atoms, or aryl; or R$^1$ and R$^2$ together comprise a methylene chain of 4 to 7 carbon atoms.

These amidated peptides and compositions containing these peptides surprisingly retain the biological activity of human thymopentin on CEM cells. These peptides are therefore inferred to produce similar in vivo biological activity in humans. A large number of these peptides may be characterized by enhanced resistance to attack by endo- and exopeptidases and trypsin-like enzymes in the digestive tract and in serum. Thus, these peptides offer significant advantages in the treatment of immune defects.

Yet a further aspect of this invention includes therapeutic compositions containing these peptides and methods for use of these peptides in treating a variety of conditions or diseases requiring immune regulation.

Other aspects and advantages of the present invention are disclosed in the following detailed description.

## Brief Description of the Drawings

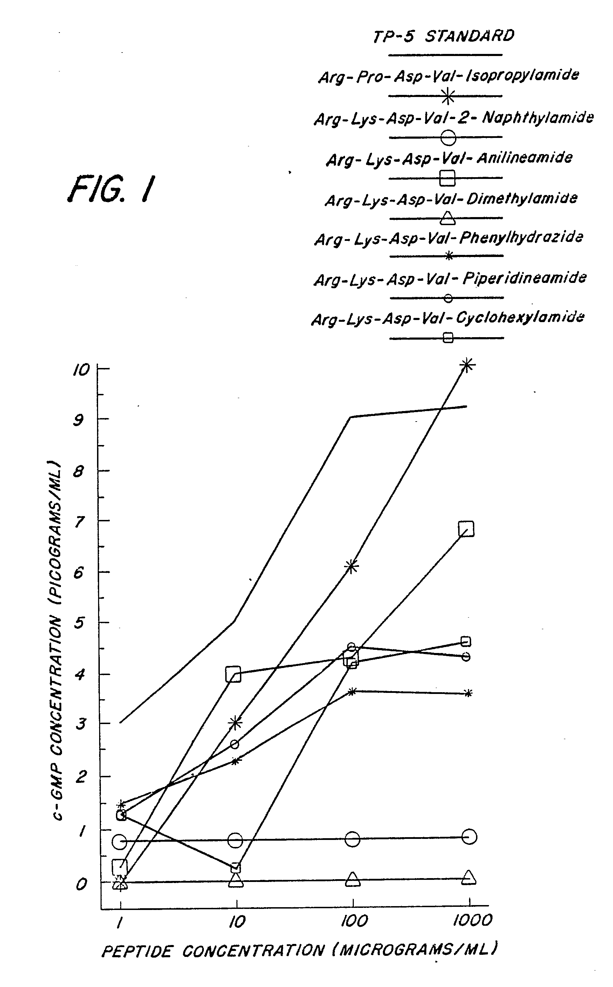

FIG. 1 is a graphical illustration of a CEM cGMP assay plotting peptide concentration (μg/ml) against cGMP levels (picogram/ml) and comparing the activity therein of thymopentin (TP-5) and peptides of this invention against controls.

## Detailed Description of the Invention

The present invention surprisingly provides compounds having T cell suppressor activity and represented by the formula R-Arg-X-Asp-Y-NR$^1$R$^2$, where R, X, Y, R$^1$ and R$^2$ are as described above.

Throughout this disclosure, the amino acid components of the peptides and certain materials used in their preparation are identified by abbreviations for convenience. Most of the three letter abbreviations for amino acids are well known. Lesser known abbreviations are Glp for pyroGlutamyl (also p-Glu) and Aib for aminoisobutyric acid. Unless otherwise indicated all amino acids are the L-isomeric configuration. Where the D-isomeric configuration is desired, it will be so indicated.

Certain preferred peptides of the present invention are those of formula wherein the second amino acid is Pro or Aib. Tetrapeptides with C terminal amides where the second amino acid X is Pro or Aib are expected to possess increased resistance to degradation by digestive and serum enzymes. Such preferred peptides include:

Arg-Pro-Asp-Val-Isopropylamide
Arg-Pro-Asp-Val-Methylamide
Acetyl-Arg-Pro-Asp-Val-Methylamide
Formyl-Arg-Pro-Asp-Val-Methylamide

Other preferred peptides of the present invention include:

Arg-Lys-Asp-Val-Anilineamide
Arg-Lys-Asp-Val-Methylamide
Arg-Lys-Asp-Val-2-Phenylhydrazide
Arg-Lys-Asp-Val-Piperidineamide
Arg-Lys-Asp-Val-Cyclohexylamide
Arg-Lys-Asp-Val-Isopropylamide

The surprising and unpredictable activity of the substituted tetrapeptides provide novel and unobvious compounds, particularly when compared to the prior art. While the tetrapeptide sequences of this invention may have been previously disclosed, e.g. the sequence Arg-Lys-Asp-Val in U. S. Patent No. 4,428,938, the present inventors have surprisingly discovered that selective amidation of these sequences at their carboxy termini provide compounds with T cell suppressor activity. Additionally, applicants have discovered that several of the peptides described above also surprisingly and unexplainably display both T cell suppressor and helper activities like thymopentin. These peptides include Arg-Pro-Asp-Val-Isopropylamide, Formyl-Arg-Pro-Asp-Val-Methylamide, and Arg-Lys-Asp-Val-Isopropylamide, as described above.

3

Thus the present inventors have provided by their teachings, novel compounds for pharmaceutical use. The selection of the particular amide groups necessary to produce active compounds of the above-disclosed tetrapeptide sequences is nowhere taught in the art, nor is the fact that the selective amidation of the present invention produces biologically active compounds from several tetrapeptides which are biologically inactive with free carboxy termini. Moreover, not all of the above-described tetrapeptide sequences, when amidated at their carboxy termini, display such T cell suppressor biological activity analogous to thymopentin. When assayed, a number of the prior art tetrapeptides when amidated are incapable of displaying such T cell suppressor activity. The compounds of the present invention are not predicted by a generalized teaching that amides of such sequences display the ability to effect the immune system or stimulate T cell helper activity. For example, the following peptides

Arg-Pro-Asp-Val-Morpholinoamide
Arg-Lys-Asp-Val-Dimethylamide
Arg-Lys-Asp-Val-2-Naphthylamide
Acetyl-Arg-Pro-Glu-Val-Ethylamide

do not display T cell suppressor stimulatory activity of thymopentin. Yet they have the same or similar peptide sequences as described in the Kisfaludy '938 patent or European Patent Application No. 89304998.1.

The peptides of this invention may generally be prepared following known techniques. Conveniently, synthetic production of the polypeptide of the invention may be according to the solid phase synthetic method or standard solution synthesis methods. For example, the solid phase synthesis method described by Merrifield in J.A.C.S, 85: 2149-2154 (1963) is well understood and is a common method for preparation of peptides. The solid phase method of synthesis involves the stepwise addition of protected amino acids to a growing peptide chain which is bound by covalent bonds to a solid resin particle. By this procedure, reagents and by-products are removed by filtration, thus eliminating the necessity of purifying intermediates. The general concept of this method depends on attachment of the first amino acid of the chain to a solid polymer by a covalent bond. Succeeding protected amino acids are added, one at a time, or in blocks, in a stepwise manner until the desired sequence is assembled. Finally, the protected peptide is removed from the solid resin support and the protecting groups are cleaved off.

The amino acids may be attached to any suitable polymer as a resin. The resin must contain a functional group to which the first protected amino acid can be firmly linked by a covalent bond. Various polymers are suitable for this purpose, such as cellulose, polyvinyl alcohol, polymethylmethacrylate, and N-alkyl benzylamine, N-alkyl benzhydrylamine and polystyrene resins. Appropriate protective groups usable in such synthesis include t-butyloxycarbonyl (BOC), benzyl (Bzl), t-amyloxycarbonyl (AOC), tosyl (Tos), o-bromo-phenylmethoxycarbonyl (BrZ), and phenylmethoxycarbonyl (Z or CBZ). Additional protective groups are identified in the above text, as well as in J.F.W. McOmie, "Protective Groups in Organic Chemistry", Plenum Press, New York, 1973. Both of these books are incorporated herein by reference.

The general procedure of preparation of the peptides of this invention involves initially attaching the protected C-terminal amino acid to the resin. After attachment the resin is filtered, washed and the protecting group (desirably BOC) on the alpha amino group of the C-terminal amino acid is removed. The removal of this protecting group must take place, of course, without breaking the bond between that amino acid and the resin. To the resulting resin peptide is then coupled the penultimate C-terminal protected amino acid. This coupling takes place by the formation of an amide bond between the free carboxy group of the second amino acid and the amino group of the first amino acid attached to the resin. This sequence of events is repeated with successive amino acids until all amino acids are attached to the resin.

Finally, the protected peptide is cleaved from the resin by an appropriate conventional technique known to those of skill in the art, e.g., aminolysis using cyclohexylamide, and the protecting groups removed to reveal the desired peptide. The cleavage techniques used to separate the peptide from the resin and to remove the protecting groups depend upon the selection of resin and protecting groups and are known to those familiar with the art of peptide synthesis.

Alternative techniques for peptide synthesis are described in "Peptide Synthesis" by Bodanszky, et al, second edition, John Wiley and Sons, 1976. For example, the peptides of the invention may also be synthesized using standard solution peptide synthesis methodologies, involving either stepwise or block coupling of amino acids or peptide fragments using chemical or enzymatic methods of amide bond formation. These solution synthesis methods are well known in the art.

The peptides of this invention have been found to exhibit biological activity similar to human thymopentin as disclosed in the above referenced U. S. patents, applications and articles. This biological activity is primarily evidenced by an assay measuring the induction of cyclic GMP production in the human T cell line CEM in comparison with human thymopentin. The induction of cGMP production by a peptide of the present invention in this assay indicates the ability of that peptide to bind to the human thymopentin receptor site on the cell and induce human thymopentin-like biological activity.

Many of the subject peptides offer further significant advantages. Many peptides of the present invention may be characterized by resistance to enzymatic degradation by either digestive or serum enzymes. Thus they demonstrate a prolonged half-life in vivo when administered by injection in a biological subject. Another advantage of many of these peptides is their capacity to be administered orally.

Because of the immunomodulatory characteristics of the subject peptides, they are therapeutically useful in the treatment of humans, and possibly animals, since they have the capability of inducing the differentiation and maturation of T cells which are capable of involvement in the immune response of the body. As a result, the subject peptides are considered to have multiple therapeutic uses.

The peptides of this invention are considered useful in assisting the collective immunity of the body, in that they will increase or assist in therapeutic stimulation of cellular immunity. The subject peptides or pharmaceutical compositions containing the peptides are generally considered to be useful in any area in which cellular immunity is an issue and particularly where there are deficiencies in immunity. They are thereby useful in the treatment of diseases involving allergic reactions, and autoimmune reactions.

Thus, where there is an excess of antibody production or cellular immunity due to unbalanced T cells, the subject peptides can correct this condition by stimulating suppressor T cell action. They are expected to be of therapeutic use in certain autoimmune diseases in which damaging antibodies are produced, such as systemic lupus erythematosus, rheumatoid arthritis, or the like.

In their broadest application, the subject peptides or pharmaceutical compounds containing same are useful for regulating the immune system of a subject, human or animal, in need of such regulation. As used herein, the term "regulate" means that the subject compounds cause the immune system to return from an abnormal, diseased state to a normal, balanced state. While this regulation may well find great application in the correction of immunological deficiencies (e.g., DiGeorge syndrome), it is also applicable to correct conditions of excess immunological activity (e.g., autoimmune diseases).

The present invention therefore includes methods for regulating the immune system of a human or animal in need of such regulation which comprises administering to said human or animal an immunoregulatorally-effective amount of at least one of the peptides as well as pharmaceutical compositions for practicing these methods.

The invention also provides a method for treatment of conditions resulting from relative or absolute deficiencies of the immune system of a subject, particularly in T cell suppressor function, which comprises administering to said subject a therapeutically-effective amount of at least one of the peptides of this invention.

As used herein, the term "therapeutically-effective amount" means an amount which is effective to treat the conditions referred to above. The invention also provides a method for inducing the differentiation and maturation of T cells which comprises administering to the subject an effective inducing amount of at least one of the peptides of the invention.

The invention further provides pharmaceutical compositions for practicing those methods. To prepare the pharmaceutical compositions of the present invention, a peptide of this invention is combined as the active ingredient in intimate admixture with a pharmaceutical carrier according to conventional pharmaceutical compounding techniques. This carrier may take a wide variety of forms depending on the form of preparation desired for administration, e.g., oral, sublingual, rectal, nasal, or parenteral.

In preparing the compositions in the preferred oral dosage form, any of the usual pharmaceutical media may be employed. For oral liquid preparations (e.g., suspensions, elixirs, and solutions), media containing, for example, water, oils, alcohols, flavoring agents, preservatives, coloring agents and the like may be used. Carriers such as starches, sugars, diluents, granulating agents, lubricants, binders, disintegrating agents, and the like may be used to prepare oral solids (e.g., powders, capsules, and tablets). Controlled release forms may also be used. Because of their ease in administration, tablets and capsules represent the most advantageous oral dosage unit form, in which case solid pharmaceutical carriers are obviously employed. If desired, tablets may be sugar coated or enteric coated by standard techniques.

For parenteral products, the carrier will usually comprise sterile water, although other ingredients, e.g., to aid solubility or for preservation purposes may be included. Injectable suspensions may also be prepared, in which case appropriate liquid carriers, suspending agents, and the like may be employed.

A peptide of the present invention is generally active when administered in amounts at about 1 μg/kg of body weight and preferably from about 0.001 to about 10 mg/kg body weight, although lower dosages may be useful. Generally, the same range of dosage amounts may be used in treatment of the diseases or conditions mentioned where immunodeficiency is to be treated. Larger amounts (e.g., about 10-100 mg/kg body weight) are useful for suppressing excess immune activity.

The following examples are presented to illustrate the invention without specifically limiting the invention thereto. In the examples and throughout the specification, parts are by weight unless otherwise indicated.

The examples employ the following abbreviations: TFA for trifluoroacetic acid; HOAc for acetic acid; $CH_2Cl_2$ for methylene chloride; $CH_3CN$ for acetonitrile; DMF for dimethyl formamide; $NH_4OAc$ for ammonium acetate; n-BuOH for n-butanol; pyr for pyridine; DCC for dicyclohexylcarbodiimide; HOBt for 1-hydroxy-benzotriazole; DMAP for dimethylaminopyridine; TCA for trichloroacetic acid; MeOH for methanol; TLC for thin layer chromatography; THF for tetrahydrofuran; EtOAc for ethylacetate; $NaHCO_3$ for sodium bicarbonate; $MgSO_4$ for magnesium sulfate; NMM for N-methylmorpholine; $Et_2O$ for diethyl ether; HPLC for high performance liquid chromatography: Pd/C for palladium on carbon; DIEA for diisopropylethylamine; iBuOCOCl for isobutyl-chloroformate; i-Pr₂O for diisopropyl ether; ONp for paranitrophenyl ester; RPMI for tissue culture medium; and PBS for phosphate buffered saline.

## Example 1. Synthesis of Arg-Pro-Asp-Val Isopropylamide

Into 40 ml $CH_2Cl_2$ was dissolved 2.25 g BOC-Pro-(Bzl)Asp-Val. The solution was chilled in an ice bath and 0.67 g HOBt and 0.90 g DCC in 4 ml DMF were added. After stirring 10 minutes, a solution of 0.30 g isopropylamine in 5 ml $CH_2Cl_2$ was added. The mixture was stirred in the ice bath for 15 miutes, then allowed to warm to room temperature. After 3 hours the precipitate was filtered. The filtrate was extracted with 10 percent citric acid solution, water, and saturated $NaHCO_3$ solution. The organic layer was dried over $MgSO_4$ and the solvent removed under reduced pressure. The product was an off-white solid weighing 2.32 g. Crystallization from EtOAc-i-Pr₂O gave 1.64 g of product, melting point 179.5-181.5°C.

To 0.84 g of the above product was added 50 ml of 40% TFA in $CH_2Cl_2$. The solution was stirred 30 minutes, then the solvent removed under reduced pressure. Addition of ether to the residue produced solid Pro-(Bzl)Asp-Val-Isopropylamide trifluoroacetate.

Into 10 ml DMF was dissolved 0.93 g BOC-arginine. The solution was chilled to -20°C and 0.18 ml NMM was added, followed by 0.22 g iBuOCOCl. The mixture was stirred at -15°C for 20 minutes, then 0.18 ml NMM and a chilled solution of the above trifluoroacetate salt in 10 ml DMF were added. After 30 minutes the cooling bath was removed and stirring continued for 2 hours. Most of the solvent was removed under reduced pressure. EtOAc and water were added to the residue. The layers were separated and the organic layer extracted with citric acid solution and twice with saturated $NaHCO_3$ solution. Solvent removal left a colorless glass weighing 1.44 g. The product was purified by flash chromatography on silica gel 60 eluted with 98:2 $CH_2Cl_2$:MeOH. The component to elute first was the desired product; pure fractions yielded 0.53 g; another 0.32 g containing a slight impurity was obtained.

The 0.53 g of purified protected tetrapeptide-amide was hydrogenated with 1 ml formic acid in 20 ml MeOH over palladium black. After one hour the mixture was filtered through Celite and washed with water. The MeOH was removed under reduced pressure. The residue was diluted with water and lyophilized, yielding 280 mg. The product was purified by chromatography on SP- Sephadex eluted with 0.30 M $NH_4OAc$ pH 5. Fractions containing the center of the main peak were combined and lyophilized to give 190 mg amorphous solid of Arg-Pro-Asp-Val-Isopropylamide.

Amino Acid Analysis: Asp, 1.01; Pro, 1.03; Val, 0.97; Arg, 1.00; 96 percent peptide.

Thin layer chromatography (Silica Gel 60)

$R_f$(I) = 0.44 (n-BuOH:HOAc:$H_2O$:Pyr/15:3:12:10)

$R_f$(II) = 0.65 (EtOAc:pyr:HOAc:$H_2O$/5:5:1:3)

$R_f$(III) = 0.16 (n-BuOH:HOAc:$H_2O$/3:1:1)

## Example 2. Synthesis of Arg-Lys-Asp-Val-Cyclohexylamide

To a solution of BOC-(Bzl)Asp-Val-ONp (1.09 g, 2.00 mmol) and cyclohexylamine (0.27 ml, 1.2 eq) in THF (10 ml) was added HOBt (0.27 g). The resulting yellow solution was stirred 24 hours and evaporated under reduced pressure. The residual oil was diluted with EtOAc and washed with saturated $NaHCO_3$, $H_2O$, 10% citric acid and saturated brine. The organic phase was dried over $MgSO_4$, filtered and evaporated to give the protected dipeptide amide.

To a stirred solution of 4.2 M HCl dioxane (10 ml) was added the protected dipeptide (0.71 g, 1.4 mmol). After 60 minutes, the reaction was evaporated under reduced pressure to give a colorless solid as the HCl salt of the dipeptide.

To a stirred solution of the HC1 salt of the dipeptide, (CBZ)₃Arg-(CBZ)-Lys-ONp (1.34 g, 1.4 mmol) and HOBt (0.19 g, 1.0 eg) in DMF (10 ml) was added NMM (0.18 ml). After 16 hours, a gelatinous precipitate had formed. The reaction mixture was quenched with saturated NaHCO₃ solution, filtered and washed with $H_2O$, 10% citric acid, $H_2O$ and $Et_2O$. Recrystallization from 4% HOAc/EtOAc gave (CBZ)₃Arg-(CBZ)-Lys-(Bzl)-Asp-Val-Cyclohexylamide as a colorless solid, 1.36 g, 73%.

The tetrapeptide (1.33 g) was deprotected in HF/anisole (30 ml/8 ml) for 60 minutes at 0°C. The residue was quenched in $Et_2O$ and filtered. The collected solids were dissolved in 10% HOAc and lyophilized to give Arg-Lys-Asp-Val-Cyclohexylamide, 460 mg.

The peptide was purified by HPLC: Whatman M-20 10/50 ODS-3 column, 10.0 ml/min flow rate, 10% $CH_3CN$, 0.01 M pH 5 $NH_4OAc$. Three injections of 150 mg in $\overline{3}$ ml buffer were used and the fraction eluting between 35.0 and 51.6 min was collected. Partial evaporation under reduced pressure and lyophilization gave the tetrapeptide amide Arg-Lys-Asp-Val-Cyclohexylamide as a colorless solid, 410 mg.

Amino Acid Analysis: Arg, 1.00; Lys, 1.00; Asp, 1.00; Val, 0.85.

Thin layer chromatography (250μ Silica Gel G)

$R_f(I)$ = 0.39 (n-BuOH:HOAc:$H_2O$:EtOAc/1:1:1:1) .

$R_f(II)$ = 0.61 (n-BuOH:HOAc:$H_2O$:Pyr/15:3:12:10)

$R_f(III)$ = 0.90 (EtOAc:HOAc:$H_2O$:Pyr/5:1:3:5)

## Example 3. Synthesis of Arg-Lys-Asp-Val-Piperidineamide

To a stirred solution of BOC-(Bzl)-Asp-Val-ONp (2.72 g, 5.00 mmol) and piperidine (0.6 ml, 1.2 eg) in THF (10 ml) was added HOBt (0.68 g, 1.0 eg). After 48 hours, the yellow solution was evaporated under reduced pressure. The oily residue was dissolved in EtOAc and washed once each with saturated NaHCO₃ solution, cold 2 M NaOH, twice with $H_2O$, once with 10% citric acid $\overline{and}$ saturated brine. The organic phase was dried over $MgSO_4$ to give a colorless oil, BOC-(Bzl)-Asp-Val-Piperidineamide, 2.36 g.

Purification by flash chromatography using MeOH:$CH_2Cl_2$ gave the protected dipeptide amide as a colorless solid, 1.65 g, 67%.

- To 4.2 M HCl-dioxane (10 ml) was added the protected dipeptide amide (1.06 g, 2.17 mmol). After 1 hour, the solution was evaporated under reduced pressure, dissolved in $CH_2Cl_2$ and re-evaporated to give the HCl salt as a colorless oil.

To a stirred solution of (CBZ)₃Arg-(CBZ)-Lys-ONp (2.08 g, 2.17 mmol), the colorless oil described above and HOBt (0.29 g) in DMF (10 ml) was added NMM (0.29 ml). After 1 hour, a precipitate began to form. After 16 hours, the reaction mixture was quenched with water. The product was extracted with EtOAc and washed with satu-

rated NaHCO₃ solution, $H_2O$, 10% citric acid, $H_2O$ and $Et_2O$. Recrystallization from HOAc/EtOAc/$Et_2O$ gave (CBZ)₃Arg-(CBZ)-Lys-(Bzl)-Asp-Val-Piperidineamide as a colorless solid, 2.40 g, 91%.

The protected tetrapeptide (1.87 g, 1.55 mmol) was slurried in $N_2$-purged 10% HOAc (100 ml) to which 10% Pd/C (0.8 g) was added. The mixture was agitated on a Parr hydrogenator (500 ml vessel, 43.5 psi). After 48 hours, the reaction mixture was filtered through a 0.45 nylon filter, partially evaporated, diluted with $H_2O$ and lyophilized to give the crude tetrapeptide as a colorless solid, 890 mg.

The peptide was purified by HPLC: Whatman M-20 10/50 ODS-3 column, 10.0 ml/min flow rate, 10% $CH_3CN$, 0.01 M, pH 5, $NH_4OAc$. Three injections of 300 mg in $\overline{3}$ ml buffer were used and the fraction eluting between 23.0 and 44.0 min was collected. Partial evaporation under reduced pressure and lyophilization gave the desired tetrapeptide amide Arg-Lys-Asp-Val- Piperidineamide as a colorless solid, 510 mg.

Amino Acid Analysis: Arg, 0.95; Lys, 1.00; Asp, 1.02; Val, 1.00; 54.2 peptide content.

Thin layer chromatography (250μ, Silica Gel G)

$R_f(I)$ = 0.31 (n-BuOH:HOAc:$H_2O$:EtOAc/1:1:1:1)

$R_f(II)$ = 0.53 (n-BuOH:HOAc:$H_2O$:Pyr/15:3:12:10)

$R_f(III)$ = 0.61 (EtOAc:HOAc:$H_2O$:Pyr/5:1:3:5)

## Example 4. Synthesis of Arg-Pro-Asp-Val-N-Methylamide

To a stirred solution of BOC-Val (10.86 g, 50.0 mmol) and DIEA, (8.70 ml, 1.0 eq), methylamine•HCl (3.38 g, 1.0 eg) and HOBT, (7.66 g, 1.0 eq) in 12:5 $CH_2Cl_2$:DMF (85 ml) was added DCC (10.32 g, 50.0 mmol). A precipitate formed. After 2 hours, the reaction was filtered and the filtrate evaporated to dryness. The oily residue was dissolved in EtOAc and washed thrice with saturated NaHCO₃ solution, $H_2O$, 10% citric acid and saturated brine. After drying over $Na_2SO_4$, the organic phase was filtered and evaporated to give a colorless solid. Triturtion in hot hexanes gave the desired amide, BOC-Val-Methylamide, 7.98 g, 69%, m.p. 120-122°C.

To this amide (4.60 g, 20.0 mmol) was added 50 TFA/$CH_2Cl_2$ (20 ml). After stirring 30 minutes, the solution was evaporated at less than 30°C, the oily residue was dissolved in $CH_2Cl_2$, washed twice with saturated NaHCO₃ solution. The organic phase was dried over $Na_2SO_4$, filtered and evaporated to give Val-Methylamide, 1.90 g, 73%.

To a stirred solution of BOC-Bzl-Asp (4.72 g, 14.6 mmol), Val-Methylamide (1.90 g, 14.6 mmol), HOBt (2.24 g, 1.0 eq) in DMF (20 ml) was added DCC (3.01 g, 14.6 mmol). A precipitate formed after 5 minutes. After 16 hours, the reaction was filtered and the filtrate quenched with half saturated $NaHCO_3$ solution.

The resulting solids were collected, washed with $H_2O$, 10% citric acid, $H_2O$, and $Et_2O$, and air-dried to give a colorless solid. Recrystallization from EtOAc gave the desired dipeptide amide, 5.85 g, 92%, BOC-(Bzl)-Asp-Val-Methylamide.

To the dipeptide amide (3.94 g, 9.05 mmol) was added 50% $TFA/CH_2Cl_2$ (20 ml). After stirring 30 minutes, the solution was evaporated at less than $30°C$ and triturated with $Et_2O$ to give the TFA dipeptide, 4.10 g, 100% as a colorless glass.

To a stirred solution of BOC-Pro (1.95 g, 9.05 mmol), the TFA dipeptide (4.10 g, 9.05 mmol) DIEA (1.73 ml, 1.1 eq), DMAP (0.12 g, 0.1 gq) in DMF (20 ml) was added DCC (1.87 g, 9.05 mmol). A precipitate formed after 5 minutes. After 16 hours, the reaction was filtered and the filtrate quenched with half saturated $NaHCO_3$ solution. The resulting solid was collected, washed with $H_2O$, 10% citric acid, $H_2O$, and $Et_2O$, and air-dried to give the desired tripeptide amide, 4.15 g, 86%. This was used without purification.

To this amide (4.15 g, 7.78 mmol) was added 50% $TFA/CH_2Cl_2$ (15 ml). After stirring 30 minutes, the solution was evaporated at less than $30°C$ and triturated with $Et_2O$ to give the TFA tripeptide, 4.25 g, 100% as a colorless solid.

To a stirred solution of 86.9 AOC-Tos-Arg (39.7 g, 7.78 mmol), the tripeptide amide (4.25 g, 7.78 mmol), NMM (0.86 ml, 1.1 eq), and HOBt (1.19 g, 1.0 eq) in DMF (15 ml) was added DCC (1.61 g, 7.78 mmol). A precipitate formed after 10 minutes. After 16 hours, the reaction was filtered and the filtrate quenched with half-saturated $NaHCO_3$ solution.

The resulting solid was collected, washed with $H_2O$, 10% citric acid, $H_2O$ ether, and air-dried to give the protected tetrapeptide amide, 4.82 g, 72%.

Half of the solid was deprotected in 50% $TFA/CH_2Cl_2$ (10 ml) for 30 minutes, evaporated under reduced pressure, and triturated with $Et_2O$. The resulting solid was cleaved with HF anisole (30 ml/8 ml) for 60 minutes at $0°C$. The residue is slurried in EtOAc and extracted twice with 10% HOAc (50 ml). The aqueous extracts are lyophilized to give crude Arg-Pro-Asp-Val-Methylamide.

The crude peptide was purified on SPC-25 Sephadex (2.6 x 83 cm column, 0.05 to 0.3 M $NH_4OAc$, pH 6.5; gradient: 100 ml/h flow rate, 9.5 ml/fraction, 206 nm detector). Fractions 113-128 were pooled and lyophilized to give 925 mg of the tetrapeptide amide, Arg-Pro-Asp-Val-Methylamide.
Amino Acid Analysis: Arg, 1.06; Pro, 0.99; Asp, 0.95; Val, 1.00; 54 peptide content.
Thin layer chromatography ($250\mu$ Silica Gel G)
$R_f(I)$ = 0.20 (n-BuOH:HOAc:$H_2O$:Upper

Phase/4:1:5)
$R_f(II)$ = 0.26 (n-BuOH:HOAc:$H_2O$:Pyr/15:3:12:10)
$R_f(III)$ = 0.26 (EtOAc:HOAc:$H_2O$:Pyr/5:1:3:5)

## Example 5. Synthesis of N-formyl-Arg-Pro-Asp-Val-Methylamide

To AOC-(Tos)-Arg-Pro-(Bzl)-Asp-Val-Methylamide (1.00 g, 1.17 mmol) was added 4.5 M HCl dioxane (10 ml). After 60 minutes, the reaction mass was evaporated, diluted with $H_2O$ and lyophilized to give the HCl tetrapeptide as a colorless powdered solid, 0.92 g, 100%.

The solid was dissolved in DMF (10 ml) and DIEA (0.93 ml, 4.0 eq), DMAP (0.08 g) and p-nitrophenyl-formate (200 mg, 1.02 eq) were added. After 2 hours, the reaction mixture was treated with 10% citric acid. The resulting solid was filtered, washed with $H_2O$ and air-dried to give the formylated peptide amide, 0.41 g.

This solid was cleaved with HF/anisole (30 ml/8 ml) for 60 minutes at $0°C$. The residue was slurried in EtOAc and extracted twice with 1% $NH_4OH$ (50 ml). The aqueous extracts were lyophilized to give the crude peptide, N-formyl-Arg-Pro-Asp-Val-Methylamide.

The crude peptide was purified on DEAE Sephadex (2.6 x 90 cm column, 0.1 M $NH_4HCO_3$, unbuffered; 100 ml/h flow water, 10 ml/fraction, 206 nm detector). Fractions 38-43 were pooled and lyophilized to give the amidated peptide 80 mg.
Amino Acid Analysis: Arg, 1.00; Pro, 1.00; Asp, 1.00; Val, 1.00; 50% peptide content
Thin layer chromatography ($250\mu$ Silica Gel G)
$R_f(I)$ = 0.32 (n-BuOH:HOAc:$H_2O$:Upper Phase/4:1:5)
$R_f(II)$ = 0.38 (EtOAc:HOAc:$H_2O$:Pyr/5:1:3:5)
$R_f(III)$ = 0.34 (Trifluoroethanol:$NH_4OH$/1:1)

## Example 6. Synthesis of Arg-Lys-Asp-Val-2-Phenylhydrazide

To a stirred solution of BOC-Val (4.34 g, 20.0 mmol) in THF (40 ml) at $-15°C$ was added NMM (2.30 ml) and then iBuOCOCl (2.64 ml). The resulting slurry was stirred 15 minutes and then phenylhydrazine (1.98 ml) was added dropwise. The slurry was stirred at -10 to $0°C$ for 1 hour and then warmed to room temperature. The reaction mixture was filtered and the filtrate evaporated under reduced pressure. The residue was dissolved in EtOAc and washed with saturated $NaHCO_3$ solution. $H_2O$ and saturated brine.

The organic phase was dried over $Na_2SO_4$, filtered and evaporated to give a yellow orange solid. Recrystallization from $CH_2Cl_2$/petroleum

ether gave BOC-Val-2-Phenylhydrazine as a colorless solid, m.p. 140-141°C, 4.96 g, 74%.

To a stirred solution of 4.2 M HCl dioxane (10 ml) was added BOC-Val-2-Phenylhydrazide (1.13 g, 3.68 mmol). After 1 hour, the reaction was evaporated under reduced pressure, triturated in $Et_2O$ and filtered to give Val-2-Phenylhydrazide the di-HCl salt; as a colorless solids 1.03 g.

To a stirred solution of the di-HCl salt (1.03 g, 3.68 mmol), BOC-(Bzl)-Asp (0.88 g) and HOBt (0.37 g) in THF (10 ml) was added NMM (0.60 ml) and then DCC (0.56 g). A precipitate formed almost at once. After 16 hours, the reaction mixture was filtered and the filtrate evaporated under reduced pressure. The residue was dissolved in EtOAc, washed with saturated $NaHCO_3$ and saturated brine. The organic phase was dried over $NaSO_4$, filtered and evaporated under reduced pressure. Recrystallization from EtOAc/hexanes gave BOC-(Bzl)-Asp-Val-2-Phenylhydrazide as a colorless solid, 1.07 g, 64%.

To a stirred solution of 4.2 M HCl dioxane (10 ml) the BOC dipeptide Phenylhydrazide (1.07 g, 1.75 mmol) was added. After 60 minutes, the solution was evaporated under reduced pressure to give a colorless oil, (Bzl)-Asp-Val-2-Phenylhydrazide hydrochloride.

To a stirred solution of $(CBZ)_3Arg$-(CBZ)Lys-ONp (1.68 g), the oil and HOBt (0.24 g) in DMF (6 ml) was added NMM (0.6 ml). After 16 hours, a gelatinous precipitate had formed. The reaction mixture was quenched with saturated $NaHCO_3$ solution and the solids were collected, washed with $H_2O$, 10% citric acid, $H_2O$ and $ET_2O$. Recrystallization from 2% HOAc/EtOAc gave $(CBZ)_3Arg$-(CBZ)-Lys-(Bzl)-Asp-Val-2-Phenylhydrazide as a colorless solid, 2.15 g, 92%.

The protected peptide (1.88 g) was cleaved in HF/anisole (30 ml/8 ml) for 60 minutes at 0°C. The residue was quenched in $ET_2O$ and filtered. The solids were extracted with 10% HOAc (100 ml) for 1 hour, filtered, and the extract lyophilized to give Arg-Lys-Asp-Val-2-Phenylhydrazide as a colorless solid, 890 mg.

The crude peptide was purified on CM Sephadex (2.6 x 88 cm column 0.3 M $NH_4OAc$, unbuffered; 100 ml/h flow rate, 12.5 ml/fraction, 225 nm detector). Fractions 270-310 were pooled and lyophilized to give 390 mg of the end product, Arg-Lys-Asp-Val-2-Phenylhydrazide.

Amino Acid Analysis: Arg, 0.98; Lys, 1.03; Asp, 1.00; Val, 0.99; 83 peptide content.

Thin layer chromatography (250 $\mu$Silica Gel G) $R_f(I)$ = 0.39 (n-BuOH:HOAc:$H_2O$:EtOAc/1:1:1:1) $R_f$-(II) = 0.51 (n-BuOH:HOAc:$H_2O$:Pyr/15:3:12:10) $R_f$-(III) = 0.59 (EtOAc:HOAc:$H_2O$:Pyr:5:1:3:5)

## Example 7. Synthesis of Arg-Lys-Asp-Val-Anilineamide

To a stirring solution of BOC-Val (4.35 g, 20.0 mmol), aniline (3.0 ml, 1.05 eq) and HOBt (3.09 g, 1.0 eq) in DMF (20 ml) at room temperature was added DCC (4.12 g, 20.0 mmol). A thick precipitate formed within 5 minutes. After 4 hours, the reaction was filtered and the filtrate poured into half-saturated $NaHCO_3$ (500 ml). A precipitate formed and was collected. The solids were washed with $H_2O$ (1:1) and air-dried. Recrystallization from hexanes/isopropanol gave the colorless solid anilide BOC-Val-Anilineamide, 3.76 g, 64%.

The BOC-Val-Anilineamide (2.50 g, 8.55 mmol) was dissolved in 50% TFA/$CH_2Cl_2$ (10 ml) and stirred for 30 minutes. The reaction mixture was evaporated under reduced pressure at room temperature and the residue dissolved in $CH_2Cl_2$. This solution was washed twice with saturated $NaHCO_3$ solution, dried over $MgSO_4$, filtered and evaporated to give the Val-Anilineamide as a light yellow oil, 1.42 g, 88%.

To a solution of BOC-(Bzl)-Asp (2.38 g, 1.0 eq), the Val-Anilineamide, and HOBt (1.13 g) in DMF (7.5 ml) was added DCC (1.52 g, 1.0 eq). A precipitate formed and the reaction was stirred for 3 hours. The mixture was filtered and the filtrate quenched with saturated $NaHCO_3$ solution. The resulting solids were collected, washed with $H_2O$ and air-dried. Recrystallization from EtOAc/petroleum ether gave the colorless BOC dipeptide amide, mp 121-122°C, 1.74 g, 46%, BOC-(Bzl)-Asp-Val-Anilineamide.

The BOC dipeptide (1.71 g, 3.44 mmol) was dissolved in 50 TFA/$CH_2Cl_2$ (10 ml) and stirred for 30 minutes. The reaction mixture was evaporated under reduced pressure at room temperature and the residue triturated in ether, filtered and air-dried.

To a stirred solution of BOC-(CBZ)-Lys (1.33 g, 3.50 mmol) in DMF (10 ml) at -20°C was added NMM (0.46 ml, 1.2 eq) and then iBuOCOCl (0.46 ml, 1.01 eq). The resulting slurry was stirred 30 minutes and the dipeptide TFA salt was then added, followed by NMM (0.46 ml). The reaction was stirred and allowed to warm to room temperature. After 1 hour, the reaction mixture was poured into half-saturated $NaHCO_3$ (200 ml). A colorless solid formed. This was collected and air-dried. Recrystallization from EtOAc/i-$Pr_2O$ gave the BOC tripeptide amide, 2.05 g, 78%, m.p. 164-167°C.

The BOC tripeptide amide (2.00 g, 2.63 mmol) was dissolved in 50% TFA/$CH_2Cl_2$ (6 ml) and stirred for 30 minutes. The reaction mixture was evaporated under reduced pressure at room temperature and the residue triturated in ether, filtered and air-dried to give the TFA salt.

To a stirred solution of $(CBZ)_3Arg$ (1.52 g, 2.63 mmol) in DMF at -20°C was added NMM (0.35 ml, 1.2 eq) and then iBuOCOCl (0.35 ml, 1.01 eq). The resulting slurry was stirred 30 minutes. NMM (0.35 ml) was then added, followed by the TFA salt. The reaction was allowed to warm to room temperature for 1 hour. The mixture was poured into saturated NaHCO₃ solution and the resulting solids were filtered, washed with H₂O and air-dried. Trituration in hot EtOH gave the protected tetrapeptide, BOC-(CBZ)-Lys-(Bzl)-Asp-Val-Anilineamide, 2.55 g, 78%.

To a stirring slurry of the tetrapeptide (2.30 g, 1.89 mmol) in trifluoroethanol (50 ml) and formic acid (97%, 5 ml) was added palladium black (0.95 g). The reaction was stirred vigorously. After 1 hour, the solution was filtered through Celite and washed with H₂O. The filtrate was partially evaporated under reduced pressure and the residue was lyophilized to give a colorless solid, 0.89 g.

The crude peptide was purified on SPC-25 Sephadex (2.6 x 89 cm column, 0.1 to 0.3 M NM₄OAc; pH 5 gradient; 100 ml/h flow rate, $\overline{15}$ ml/fraction, 206 nm detector). Fractions 253-285 were pooled and lyophilized to give 680 mg, Arg-Lys-Asp-Val-Anilineamide.

Amino Acid Analysis: Arg, 1.00; Lys, 0.98; Asp, 1.00; Val, 1.01; 100% peptide content.

Thin layer chromatography (250 $\mu$Silica Gel G)

$R_f(I)$ = 0.42 (EtOAc:HOAc:H₂O:Pyr/5:1:3:5)

$R_f(II)$ = 0.32 (n-BuOH:HOAc:H₂O:EtOAc/1:1:1:1)

$R_f(III)$ = 0.54 (n-BuOH:HOAc:H₂O:Pyr/15:3:12:10)

## Example 8. Biological Activity: Cyclic GMP Assay

This assay measures the ability of a peptide of this invention to bind to the cell membrane receptor of the intact CEM cell and selectively stimulate production of cyclic GMP, as does human thymopentin.

The CEM cell line was obtained from the American Type Culture Collection of Rockville, Md. CEM cells were freshly seeded and grown for 3 days with harvesting as described in T. Audhya et al, Arch. Biochem Biophys., 234: 167-177 (1984). The cells were washed 3 times in PBS and resuspended in RPMI 1640 at a concentration of 1.0 x 10⁷ cells/ml and were allowed to equilibrate at 37°C for 30 minutes before the addition of the test peptides (25 ul) and control peptides. The incubation was allowed to proceed in a shaking water bath for 4-5 minutes and was then terminated by addition of 1 ml ice-cold TCA (10 percent).

The cells in TCA were homogenized and sonicated to release cyclic nucleotide. The suspension was centrifuged at 3000 x g for 20 minutes at 4°C. The resulting precipitate was dissolved in 0.1 N NaOH to determine the protein content. TCA was removed from the supernatant fraction by extracting 4 times with 5 ml of water-saturated ET₂O. After the final extraction, the remaining traces of ether were removed by heating for 10 minutes in a 50°C water bath. After lyophilization the sample was reconstituted in 50 mM acetate buffer (pH 6.2) for radioimmunoassay of cyclic GMP.

For example, dose-response curves from 1 to 1000 micrograms/ml of peptide, were evaluated for each·compound. Fig. 1 shows typical dose-response curves (from a composite of 6 experiments) for active peptides Arg-Pro-Asp-Val-Isopropylamide, Arg-Lys-Asp- Val-Anilineamide, Arg-Lys-Asp-Val-Cyclohexylamide, Arg-Lys-Asp-Val-Piperidineamide and Arg-Lys-Asp-Val-Phenylhydrazide compared with thymopentin and inactive peptides, Arg-Lys-Asp-Val-Dimethylamide and Arg-Lys-Asp-Val-2-Naphthylamide in CEM cells. A threshold activity was determined for each peptide tested. This is defined as the lowest concentration of the test peptide which induced an intracellular level of cyclic GMP greater than two standard deviations above the control. The controls had intracellular cyclic GMP values of less than 0.9 picomoles/ml (mean±standard deviation). Test results were considered positive if the level of cyclic GMP was greater than 2 times that determined for the parallel negative control. Active peptides had activity thresholds ranging from 1 to 10 $\mu$g/l.

Results of the cyclic GMP assay are shown in Fig. 1 in which representative peptides of the invention have been assayed in comparison with thymopentin and control peptides. These results demonstrate the biological activity of the peptides of the invention in stimulating T cell suppressor activity in CEM cells.

## Example 9. Enzymatic Stability

To illustrate the stability of a peptide of the present invention against degradation by intestinal and serum enzymes, an exemplary peptide Arg-Pro-Asp-Val- Isopropylamide may be incubated at 37°C in rat intestinal juice and human serum for up to 120 minutes. Similarly treated for comparison are peptides such as Arg-Lys-Asp-Val-Dimethylamide and thymopentin. HPLC is expected to reveal that Arg-Lys-Asp-Val-Dimethylamide is totally digested after only a few seconds. Thymopentin is 50% degraded under these conditions at about 20 seconds. The peptides of this invention are expected to remain substantially undegraded in both serum and intestinal juice for considerably longer than the·thymopentin or a control peptide.

Example 10. Synthesis of Arg-Lys-Asp-Val-Methylamide

A. Nα-t-butyloxycarbonyl-(N-benzyloxycarbonyl) Lysyl-(β-benzyl)Aspartyl-Valine methyl amide

Into 50 ml 85 percent acetic acid was dissolved 3.21 g Nα-t-butyloxycarbonyl-(N-benzyloxycarbonyl(Lysyl-(β-benzyl)Aspartyl-Valine phenacyl ester. Zinc dust, 5.2 g, was added and the mixture stirred vigorously for one hour. The reaction mixture was filtered, the filtrate diluted with water and extracted three times with ethyl acetate. The combined organic layers were extracted with water and saturated sodium chloride solution, then dried over anhydrous magnesium sulfate. Solvent removal left an oil. Addition of ether, then hexane produced a gummy precipitate. This material was triturated with hexane until it was a dry white solid, weighing 1.75 g. The product was dissolved in 14 ml ethyl acetate and 1 ml dimethylformamide.

After chilling the solution to -20°C, 0.30 ml N-methylmorpholine was added, followed by 0.36 g isobutylchloroformate dropwise. The reaction mixture was stirred at -15°C for 20 minutes, then 0.29 ml N-methylmorpholine and a chilled solution of 0.18 g methylamine hydrochloride in 10 ml 95 percent ethanol was added. A thick precipitate formed. 5 ml DMF was added to facilitate stirring and the mixture stirred at 0°C for 90 minutes, then at room temperature for 30 minutes. The reaction mixture was added to 75 ml water.

The organic phase became globules of waxy solid on stirring the mixture. The solid was filtered and the filtrate extracted with ethyl acetate. The residue from evaporation of the organic phase was combined with the solid product and it was crystallized from ethyl acetate-isopropyl ether. The yield was 1.53 g, melting point 183.5-184.5°C. Elemental analysis: C-61.57 (61.96), H-7.21 (7.37), N-9.81 (10.04).

B. Tri benzyloxycarbonyl-Arginyl-(N-benzyloxycarbonyl)Lysyl-(β-benzyl)Asp-artyl-Valine methyl amide

To 1.46 g of the product of part A above was added 50 ml of 50 percent trifluoroacetic acid in methylene chloride. After stirring for 25 minutes, the solvent was evaporated and ether added to the residue, producing a white solid. This material was filtered and washed with ether.

Tribenzyloxycarbonyl-Arginine 1.33 g was dissolved in 10 ml DMF. The solution was chilled to -20°C and 0.25 ml N-methylmorpholine was added followed by 0.31 g isobutylchloroformate in 2 ml DMF. The mixture was stirred at -20°C for 20 minutes, then 0.26 ml N-methylmorpholine and a chilled solution of the tripeptide trifluoroacetate in 10 ml DMF were added. The reaction mixture was stirred at -20°C to -10°C for 30 minutes, then at room temperature overnight. After 16 hours, the mixture was added to 125 ml water. The precipitate was collected by filtration and washed with warm methanol and ethyl acetate. The yield was 1.74 g with a melting point 118-120°C. Amino acid analysis: Asp-0.99, Val-1.03, Tyr-0.99, Lys-0.98, Arg-1.00; 67.7 percent peptide.

C. Arginyl-Lysyl-Aspartyl-Valine methyl amide

Deprotection of 1.72 g of the product of part B above was accomplished by reduction with 2 ml formic acid over 1 g palladium black in 40 ml trifluoroethanol. The mixture was stirred rapidly for 1 1/2 hours in a flask stoppered with a balloon. The palladium was removed by filtration through Celite, washing with water. Most of the trifluoroethanol was removed from the filtrate by rotary evaporation. The residue was diluted with 5 percent acetic acid and lyophilized. The product weighed 676 mg.

The peptide was purified by chromatography on a 2.6 x 85 cm column of SP-Sephadex eluted with a gradient of 0.20-0.50N NH₄OAc pH 5.5 over 3L. Fractions of 10 ml were collected, monitoring at 206 nm. The major peak appeared in the fractions 185-220; three cuts were taken: 185-190, 191-205 and 206-220. Lyophilization yielded 21 mg, 497 mg, and 235 mg, respectively. The first cut contained an impurity, the other two were greater than 99.5 pure.

HPLC, μ-Bondapak $C_{18}$: rt = 8.5 min with 0.01M NH₄OAc pH 5 at 2.0 ml/min. Amino Acid Analysis: Asp-1.01, Val-0.95, Lys-1.01, Arg-1.02; 56 percent peptide. Thin layer chromatography (silica gel 60)

$R_f$ = 0.19 (n-BuOH:HOAc:H₂O:pyridine/15:3:12:10)
$R_f$ = 0.15 (EtOAc:pyr:H₂O 5:5:1:3)
$R_f$ = 0.45 (TFA:NH₄OH 2:1)

Example 11. Synthesis of Arg-Lys-Asp-Val-2-propylamide

A. α-Boc-ε-(Cbz)-Lys-(β-Bzl)Asp-Val

To a rapidly stirred solution of α-Boc-ε-(Cbz)-Lys-(β-Bzl)Asp-Val phenacyl ester (8.02 g, 10.0 mmol) in 85 HOAc (100 ml) at room temperature was added acid-etched zinc dust (12.4 g). After 1 hour, the reaction was filtered, and the filtrate was evaported in vacuo. The resulting oily residue was triturated in Et₂O. The solids which formed were

collected, washed with $Et_2O$ and $H_2O$ and dried in vacuo at $40°C$ to give 4.95 g, 72%.

## ∞. α-Boc-ε-(Cbz)Lys-(β-Bzl)-Asp-Val-NHCH(CH₃)₂

To a stirred solution of the Boc tripeptide (1.00 g, 1.46 mmol in THF (5 ml) at $-15°C$ was added NMM (170 μl, 1.06 eq) and the iBuOCOCl (195 μl, 1.03 eq). The resulting faintly turbid solution was stirred 15 minutes and then 2-propylamine (150 μl, 1.1 eq) was added. The reaction was warmed to $-5°C$. A gelatinous solid formed and the reaction was then allowed to warm to room temperature. After 16 hours, the reaction was evaporated in vacuo at $30°C$ and the solid residue was washed with 10% citric acid, $H_2O$, saturated $NaHCO_3$ solution and $H_2O$. Air-drying gave the colorless Boc tripeptide amide, 2.58 g, 55%.

## C. (Cbz)₃Aro-ε-(Cbz)Lys-(β-Bzl)Asp-Val-NHCH-(CH₃)₂

The Boc tripeptide amide (0.55 g, 0.76 mmol) was dissolved in 4.4 M HCl dioxane (5 ml) and stirred for 45 minutes. The solution was evaporated in vacuo, and the solid triturated in $Et_2O$. Filtration gave the colorless HCl tripeptide amide, 0.51 g, 100%.

To a stirred solution of HCl tripeptide amide, (Cbz)₃Arg p-nitrophenylester (0.53 g, 1.0 eq) and HOBT (0.12 g, 1.0 eq) in DMF (5 ml) at room temperature was added NMM (92 μl, 1.1 eq). A precipitate slowly formed over 16 hours. The gelatinous reaction mixture was stirred with saturated $NaHCO_3$ solution and filtered. The solid residue was washed with $H_2O$, 10% citric acid, $H_2O$ and $Et_2O$. The resulting protected tetrapeptide amide was recrystallized from 1 HOAc/EtOAc to give a colorless solid, 743 mg, 83%.

## D. Arg-Lys-Asp-Val-NHCH(CH₃)₂

The protected tetrapeptide (0.70 g, 0.59 mmol) was slurried in 90% HOAc (50 ml). The mixture was purged with $N_2$ and charged with 10% Pd-C (0.4 g). Hydrogenation in a Parr shaker apparatus (250 ml vessel, $P_0$ = 52.0 psi) for 64 hours, filtration through a 0.45 μ nylon filter, partial evaporation and lyophilization gave the crude tetrapeptide, 385 mg.

The crude peptide was purified on CM Sephadex (2.6 x 93 cm column, 1.7 l, 0.2 M, then 0.3 M NH₄OAc, unbuffered; 100 ml/hour flow rate, 10 ml fraction, 225 nm detector). Fractions 335-370 were pooled and lyophilized to give 231 mg.

Amino Acid Analysis: Arg-1.00, Lys-1.02, Asp-0.98, Val-1.00; 80.3 peptide content.
Thin layer chromatography (250 μ, Silica Gel G)
$R_f$ = 0.24 (n-BuOH:HOAc:H₂O:EtOAc/1:1:1:1)
$R_f$ = 0.52 (n-BuOH:HOAc:H₂O:pyridine/15:3:12:10)
$R_f$ = 0.61 (EtOAc:HOAc:H₂O:pyridine/5:1:3:5)

## Example 12. Synthesis of N-Acetyl-Arg-Pro-Asp-Val-N-methylamide

To Aoc#(Tos)⁹Arg-Pro-(β-Bzl)Asp-ValNH₃ (1.50 g, 1.75 mmol) was added 4.5 M HCl dioxane (10 ml). After 45 min, the reaction mass was evaporated, diluted with $H_2O$ and lyophilized to give the HCl tetrapeptide as a colorless powdered solid, 1.38 g, 99%.

The solid was dissolved in DMF (10 ml) and DIEA (1.4 ml, 4 eq), DMAP (0.11 g), and $Ac_2O$ (1.0 ml) were added. After 1 hour, the reaction mixture was treated with 10 citric acid. The resulting solid was filtered, washed with $H_2O$ and air-dried. Trituration in hot EtOAc gave the protected acetyl tetrapeptide, 1.42 g, 100%.

This solid was cleaved with HF/anisole (30 ml/8 ml) for 60 minutes at $0°C$. The residue is slurried in EtOAc and extracted twice with 10 HOAc (50 ml). The aqueous extracts are lyophilized to give crude Ac-Arg-Pro-Asp-ValNHCH₃.

The crude peptide was purified on DEAE Sephadex (2.6 x 90 cm column, 0.1 M NH₄HCO₃, unbuffered; 100 ml/hour flow rate, 10 ml/fraction 206 nm detector). Fractions 32-40 were pooled and lyophilized to give 345 mg, 32%.
Amino Acid Analysis: Arg-0.98, Pro-0.97, Asp-1.00, Val-1.05; 87.0 peptide content.
Thin layer chromatography (250 μ Silica Gel G)
$R_f$ = 0.39 (n-BuOH:HOAc:H₂O:EtOAc/1:1:1:1)
$R_f$ = 0.56 (n-BuOH:HOAc:H₂O:pyridine/15:3:12:10)
$R_f$ = 0.76 (EtOAc:HOAc:H₂O:pyridine/5:1:3:5)

The above examples have been presented for illustrative purposes only and do not limit the scope of the present invention, which is set out in the following claims.

## Claims

1. A peptide having the ability to increase cGMP activity in human T cell line CEM and selected from peptides of the formula
R-Arg-X-Asp-Y-NR¹R²
wherein
R is H, lower alkyl, acetyl, formyl, lower alkanoyl;
X is Pro, dehydro-Pro, hydroxyl-Pro, D-Lys, Aib or Lys;
Y is Gly or a D or L form of an amino acid selected from Val, Ala, Ile, Leu, Asp, Glu, Gln or Lys;

$R^1$ is H and $R^2$ is lower alkyl or alkenyl having 1 to 10 carbon atoms, cyclic alkyl or alkenyl having 4 to 8 carbon atoms, aryl or $NHR^3$, where $R^3$ is a lower alkyl having 1 to 10 carbon atoms, cyclic alkyl or alkenyl having 4 to 8 carbon atoms, or aryl; or $R^1$ and $R^2$ together comprise a methylene chain of 4 to 7 carbon atoms.

2. A peptide according to claim 1, characterised in that X is Pro or Lys, and Y is Val.

3. A peptide according to claim 1, characterised in that it is selected from
Arg-Pro-Asp-Val-Isopropylamide,
Arg-Lys-Asp-Val-Anilineamide,
Formyl-Arg-Pro-Asp-Val-Methylamide,
Acetyl-Arg-Pro-Asp-Val-Methylamide,
Arg-Pro-Asp-Val-Methylamide,
Arg-Lys-Asp-Val-Methylamide,
Arg-Lys-Asp-Val-2-Phenylhydrazide,
Arg-Lys-Asp-Val-Piperidineamide,
Arg-Lys-Asp-Val-Cyclohexylamide, and
Arg-Lys-Asp-Val-Isopropylamide.

4. A pharmaceutical composition comprising a therapeutically effective amount of at least one peptide according to any of claims 1 to 3 in a pharmaceutically acceptable formulation.

5. A composition according to claim 4, characterised in that it is in a form suitable for oral administration.

6. A process for producing a peptide according to any of claims 1 to 3, characterised in that the peptide is synthesized in solid phase.

7. A process for preparing a peptide according to any of claims 1 to 3, characterised in that the peptide is synthesized in solution.

8. The use of a peptide according to any of claims 1 to 3 in the manufacture of a pharmaceutical composition for regulating the immune system of a human.

9. The use of a peptide according to any of claims 1 to 3 in the manufacture of a pharmaceutical composition for regulating a deficiency or excess of T cell function in a human.

10. A method for regulating in a subject a deficiency or excess of T-cell function comprising administering to said subject a therapeutically effective amount of at least one peptide according to any of claims 1 to 3.

FIG. 1

TP-5 STANDARD
——————

Arg-Pro-Asp-Val-Isopropylamide
———— * ————

Arg-Lys-Asp-Val-2-Naphthylamide
——— ○ ———

Arg-Lys-Asp-Val-Anilineamide
——— ☐ ———

Arg-Lys-Asp-Val-Dimethylamide
——— △ ———

Arg-Lys-Asp-Val-Phenylhydrazide
——— * ———

Arg-Lys-Asp-Val-Piperidineamide
——— ⊖ ———

Arg-Lys-Asp-Val-Cyclohexylamide
——— ⊟ ———

c-GMP CONCENTRATION (PICOGRAMS/ML)

PEPTIDE CONCENTRATION (MICROGRAMS/ML)